# FASCICULE DE BREVET EUROPEEN

(11) **EP 1 651 762 B1**
(45) Date de publication et mention de la délivrance du brevet: **20.11.2013**
(21) Numéro de dépôt: 04767568.1
(22) Date de dépôt: 02.07.2004
(51) Int. Cl.: A61K 38/00, C12N 15/11, C12N 15/63, A61K 31/713

(54) **PETITS ARN INTERFERENTS SPECIFIQUES DES SOUS-UNITES ALPHA, ALPHA' ET ß DE LA PROTEINE KINASE CK2 ET LEURS APPLICATIONS**
FÜR DIE UNTEREINHEITEN ALPHA, ALPHA' UND BETA DES KINASEPROTEINS CK2 SPEZIFISCHE SIRNA SOWIE ANWENDUNGEN DAVON
SMALL INTERFERING RNA SPECIFIC TO SUB-UNITS ALPHA, ALPHA' AND BETA OF THE KINASE PROTEIN CK2, AND THE APPLICATIONS OF THE SAME

(30) Priorité: 02.07.2003 FR 0308032
(43) Date de publication de la demande: 03.05.2006
(73) Titulaire: Commissariat à l'Énergie Atomique et aux Énergies Alternatives, 75015 Paris (FR); INSTITUT NATIONAL DE LA SANTE ET DE LA RECHERCHE MEDICALE, 75654 Paris Cedex 13 (FR)
(72) Inventeur: SCHAACK, Béatrice, F-38000 Grenoble (FR); COCHET, Claude, F-38640 Claix (FR); FILHOL-COCHET, Odile, F-38640 Claix (FR); FOUQUE, Brigitte, F-38170 Seyssinet Pariset (FR)
(74) Mandataire: Vialle-Presles, Marie José
(86) Numéro de dépôt international: PCT/FR2004/001729
(87) Numéro de publication internationale: WO 2005/005632

(56) Documents cités:
- WO-A-00/63364
- US-A1- 2002 147 163
- US-B1- 6 455 307
- ULLOA L ET AL: "DEPLETION OF CASEIN KINASE II BY ANTISENSE OLIGONUCLEOTIDE PREVENTS NEURITOGENESIS IN NEUROBLASTOMA CELLS" EMBO JOURNAL, OXFORD UNIVERSITY PRESS, SURREY, GB, vol. 12, no. 4, 1993, pages 1633-1640, XP002952118 ISSN: 0261-4189 cité dans la demande
- FAUST RUSSELL A ET AL: "Antisense oligonucleotides against protein kinase CK2-alpha inhibit growth of squamous cell carcinoma of the head and neck in vitro" HEAD AND NECK, vol. 22, no. 4, juillet 2000 (2000-07), pages 341-346, XP009027373 ISSN: 1043-3074 cité dans la demande
- VOORHOEVE P M ET AL: "Knockdown stands up" TRENDS IN BIOTECHNOLOGY, ELSEVIER PUBLICATIONS, CAMBRIDGE, GB, vol. 21, no. 1, janvier 2003 (2003-01), pages 2-4, XP004397628 ISSN: 0167-7799 cité dans la demande
- READ M L ET AL: "RNA-based therapeutic strategies for cancer" EXPERT OPINION ON THERAPEUTIC PATENTS 01 MAY 2003 UNITED KINGDOM, vol. 13, no. 5, 1 mai 2003 (2003-05-01), pages 627-638, XP002314125 ISSN: 1354-3776
- PARRISH S ET AL: "Functional anatomy of a dsRNA trigger: Differential requirement for the two trigger strands in RNA interference" MOLECULAR CELL, CELL PRESS, CAMBRIDGE, MA, US, vol. 6, no. 5, novembre 2000 (2000-11), pages 1077-1087, XP002226298 ISSN: 1097-2765
- ELBASHIR S M ET AL: "Functional anatomy of siRNAs for mediating efficient RNAi in Drosophila melanogaster embryo lysate" EMBO JOURNAL, OXFORD UNIVERSITY PRESS, SURREY, GB, vol. 20, no. 23, 3 décembre 2001 (2001-12-03), pages 6877-6888, XP002225998 ISSN: 0261-4189
- BRANTL S: "Antisense-RNA regulation and RNA interference" BIOCHIMICA ET BIOPHYSICA ACTA . GENE STRUCTURE AND EXPRESSION, ELSEVIER, AMSTERDAM, NL, vol. 1575, no. 1-3, 3 mai 2002 (2002-05-03), pages 15-25, XP004356720 ISSN: 0167-4781
- BUCHOU THIERRY ET AL: "La proteine kinase CK2, une enzyme qui cultive la difference." M-S (MEDECINE SCIENCES), vol. 19, no. 6-7, juin 2003 (2003-06), - juillet 2003 (2003-07) pages 709-716, XP009027436 ISSN: 0767-0974
- AHMED KHALIL ET AL: "Joining the cell survival squad: An emerging role for protein kinase CK2" TRENDS IN CELL BIOLOGY, vol. 12, no. 5, mai 2002 (2002-05), pages 226-230, XP002273461 ISSN: 0962-8924
- VALERO EMMANUELLE ET AL: "Modulation of the protein kinase CK2 activity by a synthetic peptide corresponding to the N-terminus of its beta regulatory subunit" BIOCHEMICAL AND BIOPHYSICAL RESEARCH COMMUNICATIONS, vol. 232, no. 1, 1997, pages 178-182, XP002273462 ISSN: 0006-291X

### SEQUENCE LISTING

<110> COMMISSARIAT A L'ENERGIE ATOMIQUE
   INSTITUT NATIONAL DE LA SANTE ET DE LA RECHERCHE MEDICALE
   SCHAACK, Béatrice
   COCHET, Claude
   FILHOL-COCHET, Odile
   FOUQUE, Brigitte
<120> PETITS ARN INTERFERENTS SPECIFIQUES DES SOUS-UNITES ALPHA, ALPHA', ET
   BETA DE LA PROTEINE KINASE CK2 ET LEURS APPLICATIONS
<130> F263PCT96
<150> FR0308032 <151> 2003-07-02
<160> 87
<170> PatentIn version 3.1
<210> 1
   <211> 21
   <212> DNA
   <213> mus musculus
<400> 1
   aagcagggcc agagtttaca c 21
<210> 2
   <211> 21
   <212> DNA
   <213> Mus musculus
<400> 2
   aacacacaca gaccccgaga g 21
<210> 3
   <211> 21
   <212> DNA
   <213> Mus musculus
<400> 3
   cagaccccga gagtactggg a 21
<210> 4
   <211> 21
   <212> DNA
   <213> Mus musculus
<400> 4
   aatttgagag gtgggcccaa c 21
<210> 5
   <211> 21
   <212> DNA
   <213> Mus musculus
<400> 5
   aatgtccgag ttgcttctcg a 21
<210> 6
   <211> 21
   <212> DNA
   <213> Mus musculus
<400> 6
   tgtggagctt gggttgtatg c 21
<210> 7
   <211> 20
   <212> DNA
   <213> Mus musculus
<400> 7
   tcagttggtg aggatagcca 20
<210> 8
   <211> 21
   <212> DNA
   <213> Mus musculus
<400> 8
   tggtgaggat agccaaggtt c 21
<210> 9
   <211> 19
   <212> DNA
   <213> Mus musculus
<400> 9
   aggatagcca aggttctgg 19
<210> 10
   <211> 21
   <212> DNA
   <213> Mus musculus
<400> 10
   aacgatatct tgggcagaca c 21
<210> 11
   <211> 21
   <212> DNA
   <213> Mus musculus
<400> 11
   gatatcttgg gcagacactc c 21
<210> 12
   <211> 21
   <212> DNA
   <213> Mus musculus
<400> 12
   aaaaccagca tcttgtcagc c 21
<210> 13
   <211> 21
   <212> DNA
   <213> Mus musculus
<400> 13
   aaccagcatc ttgtcagccc t 21
<210> 14
   <211> 21
   <212> DNA
   <213> Homo sapiens
<400> 14
   aacagtctga ggagccgcga g 21
<210> 15
   <211> 21
   <212> DNA
   <213> Homo sapiens
<400> 15
   aaaacttggt cggggcaagt a 21
<210> 16
   <211> 21
   <212> DNA
   <213> Homo sapiens
<400> 16
   aaaggaccct gtgtcaaaga c 21
<210> 17
   <211> 21
   <212> DNA
   <213> Homo sapiens
<400> 17
   aagcaactct accagatcct g 21
<210> 18
   <211> 21
   <212> DNA
   <213> Homo sapiens
<400> 18
   aaagctctgg attactgcca c 21
<210> 19
   <211> 21
   <212> DNA
   <213> Homo sapiens
<400> 19
   aagggaatca tgcacaggga t 21
<210> 20
   <211> 21
   <212> DNA
   <213> Homo sapiens
<400> 20
   aagggaccag agctccttgt g 21
<210> 21
   <211> 21
   <212> DNA
   <213> Homo sapiens
<400> 21
   aattgccaag gttctgggga c 21
<210> 22
   <211> 21
   <212> DNA
   <213> Homo sapiens
<400> 22
   aacattcacg gaagcgctgg g 21
<210> 23
   <211> 21
   <212> DNA
   <213> Homo sapiens
<400> 23
   aacaggcacc ttgtcagccc g 21
<210> 24
   <211> 21
   <212> DNA
   <213> Homo sapiens
<400> 24
   aaagaggcca tggagcaccc a 21
<210> 25
   <211> 21
   <212> DNA
   <213> Homo sapiens
<400> 25
   aaggagcagt cccagccttg t 21
<210> 26
   <211> 20
   <212> DNA
   <213> Homo sapiens
<400> 26
   aagactacat ccaggacaat 20
<210> 27
   <211> 21
   <212> DNA
   <213> Homo sapiens
<400> 27
   tcaatgagca ggtccctcac t 21
<210> 28
   <211> 21
   <212> DNA
   <213> Homo sapiens
<400> 28
   caatgagcag gtccctcact a 21
<210> 29
   <211> 21
   <212> DNA
   <213> Homo sapiens
<400> 29
   acctggagcc tgatgaagaa c 21
<210> 30
   <211> 21
   <212> DNA
   <213> Homo sapiens
<400> 30
   tggagcctga tgaagaactg g 21
<210> 31
   <211> 21
   <212> DNA
   <213> Homo sapiens
<400> 31
   ggagcctgat gaagaactgg a 21
<210> 32
   <211> 21
   <212> DNA
   <213> Homo sapiens
<400> 32
   aagacaaccc caaccagagt g 21
<210> 33
   <211> 21
   <212> DNA
   <213> Homo sapiens
<400> 33
   cctgtcggac atcccaggtg a 21
<210> 34
   <211> 21
   <212> DNA
   <213> Homo sapiens
<400> 34
   aagctctact gccccaagtg c 21
<210> 35
   <211> 21
   <212> DNA
   <213> Homo sapiens
<400> 35
   ccaagagacc tgccaaccag t 21
<210> 36
   <211> 21
   <212> DNA
   <213> Homo sapiens
<400> 36
   ccaggctcta cggtttcaag a 21
<210> 37
   <211> 21
   <212> DNA
   <213> Homo sapiens
<400> 37
   aagatccatc cgatggccta c 21
<210> 38
   <211> 21
   <212> DNA
   <213> Homo sapiens
<400> 38
   agcaacttca agagcccagt c 21
<210> 39
   <211> 21
   <212> DNA
   <213> Homo sapiens
<400> 39
   aacttcaaga gcccagtcaa g 21
<210> 40
   <211> 21
   <212> DNA
   <213> Homo sapiens
<400> 40
   agagcccagt caagacgatt c 21
<210> 41
   <211> 21
   <212> DNA
   <213> Artificial sequence
<220>
   <223> brin sens siRNA
<400> 41
   gcagggccag aguuuacact t 21
<210> 42
   <211> 21
   <212> DNA
   <213> Artificial sequence
<220>
   <223> brin sens siRNA
<400> 42
   cacacacaga ccccgagagt t 21
<210> 43
   <211> 21
   <212> DNA
   <213> Artificial sequence
<220>
   <223> brin sens siRNA
<400> 43
   aauacacaca gaccucgagt t 21
<210> 44
   <211> 21
   <212> DNA
   <213> Artificial sequence
<220>
   <223> brin sens siRNA
<400> 44
   gaccccgaga guacugggat t 21
<210> 45
   <211> 21
   <212> DNA
   <213> Artificial sequence
<220>
   <223> brin sens siRNA
<400> 45
   uuugagaggu gggcccaact t 21
<210> 46
   <211> 21
   <212> DNA
   <213> Artificial sequence
<220>
   <223> brin sens siRNA
<400> 46
   uguccgaguu gcuucucgat t 21
<210> 47
   <211> 21
   <212> DNA
   <213> Artificial sequence
<220>
   <223> brin sens siRNA
<400> 47
   uggagcuugg guuguaugct t 21
<210> 48
   <211> 21
   <212> DNA
   <213> Artificial sequence
<220>
   <223> brin sens siRNA
<400> 48
   caguugguga ggauagccat t 21
<210> 49
   <211> 21
   <212> DNA
   <213> Artificial sequence
<220>
   <223> brin sens siRNA
<400> 49
   gugaggauag ccaagguuct t 21
<210> 50
   <211> 21
   <212> DNA
   <213> Artificial sequence
<220>
   <223> brin sens siRNA
<400> 50
   aggauagcca agguucuggt t 21
<210> 51
   <211> 21
   <212> DNA
   <213> Artificial sequence
<220>
   <223> brin sens siRNA
<400> 51
   cgauaucuug ggcagacact t 21
<210> 52
   <211> 21
   <212> DNA
   <213> Artificial sequence
<220>
   <223> brin sens SiRNA
<400> 52
   uaucuugggc agacacucct t 21
<210> 53
   <211> 21
   <212> DNA
   <213> Artificial sequence
<220>
   <223> brin sens siRNA
<400> 53
   aaccagcacc uugucagcct t 21
<210> 54
   <211> 21
   <212> DNA
   <213> Artificial sequence
<220>
   <223> brin sens siRNA
<400> 54
   ccagcaccuu gucagcccut t 21
<210> 55
   <211> 21
   <212> DNA
   <213> Artificial sequence
<220>
   <223> brin sens siRNA
<400> 55
   cagccugagg agccgcgagt t 21
<210> 56
   <211> 21
   <212> DNA
   <213> Artificial sequence
<220>
   <223> brin sens siRNA
<400> 56
   aacuuggucg gggcaaguat t 21
<210> 57
   <211> 21
   <212> DNA
   <213> Artificial sequence
<220>
   <223> brin sens siRNA
<400> 57
   aggacccugu gucaaagact t 21
<210> 58
   <211> 21
   <212> DNA
   <213> Artificial sequence
<220>
   <223> brin sens siRNA
<400> 58
   gcaacucuac cagauccugt t 21
<210> 59
   <211> 21
   <212> DNA
   <213> Artificial sequence
<220>
   <223> brin sens siRNA
<400> 59
   agcucuggau uacugccact t 21
<210> 60
   <211> 21
   <212> DNA
   <213> Artificial sequence
<220>
   <223> brin sens siRNA
<400> 60
   gggaaucaug cacagggaut t 21
<210> 61
   <211> 21
   <212> DNA
   <213> Artificial sequence
<220>
   <223> brin sens siRNA
<400> 61
   gggaccagag cuccuugugt t 21
<210> 62
   <211> 21
   <212> DNA
   <213> Artificial sequence
<220>
   <223> brin sens siRNA
<400> 62
   uugccaaggu ucuggggact t 21
<210> 63
   <211> 21
   <212> DNA
   <213> Artificial sequence
<220>
   <223> brin sens siRNA
<400> 63
   cauucacgga agcgcugggt t 21
<210> 64
   <211> 21
   <212> DNA
   <213> Artificial sequence
<220>
   <223> brin sens siRNA
<400> 64
   caggcaccuu gucagcccgt t 21
<210> 65
   <211> 21
   <212> DNA
   <213> Artificial sequence
<220>
   <223> brin sens siRNA
<400> 65
   agaggccaug gagcacccat t 21
<210> 66
   <211> 21
   <212> DNA
   <213> Artificial sequence
<220>
   <223> brin sens siRNA
<400> 66
   ggagcagucc cagccuugut t 21
<210> 67
   <211> 20
   <212> DNA
   <213> Artificial sequence
<220>
   <223> brin sens siRNA
<400> 67
   gacuacaucc aggacaautt 20
<210> 68
   <211> 19
   <212> DNA
   <213> Artificial sequence
<220>
   <223> brin sens siRNA
<400> 68
   aaugagcagg ucccucacu 19
<210> 69
   <211> 21
   <212> DNA
   <213> Artificial sequence
<220>
   <223> brin sens siRNA
<400> 69
   caaugagcag gucccucacu a 21
<210> 70
   <211> 21
   <212> DNA
   <213> Artificial sequence
<220>
   <223> brin sens siRNA
<400> 70
   accuggagcc ugaugaagaa c 21
<210> 71
   <211> 21
   <212> DNA
   <213> Artificial sequence
<220>
   <223> brin sens siRNA
<400> 71
   uggagccuga ugaagaacug g 21
<210> 72
   <211> 21
   <212> DNA
   <213> Artificial sequence
<220>
   <223> brin sens siRNA
<400> 72
   ggagccugau gaagaacugg a 21
<210> 73
   <211> 21
   <212> DNA
   <213> Artificial sequence
<220>
   <223> brin sens siRNA
<400> 73
   aagacaaccc caaccagagu g 21
<210> 74
   <211> 21
   <212> DNA
   <213> Artificial sequence
<220>
   <223> brin sens siRNA
<400> 74
   ccugucggac aucccaggug a 21
<210> 75
   <211> 21
   <212> DNA
   <213> Artificial sequence
<220>
   <223> brin sens siRNA
<400> 75
   gcucuacugc cccaagugct t 21
<210> 76
   <211> 21
   <212> DNA
   <213> Artificial sequence
<220>
   <223> brin sens siRNA
<400> 76
   ccaagagacc ugccaaccag u 21
<210> 77
   <211> 21
   <212> DNA
   <213> Artificial sequence
<220>
   <223> brin sens siRNA
<400> 77
   ccaggctcta cggtttcaag a 21
<210> 78
   <211> 21
   <212> DNA
   <213> Artificial sequence
<220>
   <223> brin sens siRNA
<400> 78
   gauccauccg auggccuact t 21
<210> 79
   <211> 21
   <212> DNA
   <213> Artificial sequence
<220>
   <223> brin sens siRNA
<400> 79
   agcaacuuca agagcccagu c 21
<210> 80
   <211> 21
   <212> DNA
   <213> Artificial sequence
<220>
   <223> brin sens siRNA
<400> 80
   aacttcaaga gcccagtcaa g 21
<210> 81
   <211> 21
   <212> DNA
   <213> Artificial sequence
<220>
   <223> brin sens siRNA
<400> 81
   agagcccagt caagacgatt c 21
<210> 82
   <211> 64
   <212> DNA
   <213> Artificial
<400> 82
   gatcccctga agactacatc caggacttca agagagtcct ggatgtagtc ttcatttttg 60
   gaaa 64
<210> 83
   <211> 21
   <212> DNA
   <213> ARTIFICIAL SEQUENCE
<220>
   <223> brin sens siRNA
<400> 83
   aagacuacau ccaggacaat t 21
<210> 84
   <211> 21
   <212> DNA
   <213> artificial sequence
<220>
   <223> brin antisens siRNA
<400> 84
   uuguccugga uguagucuut t 21
<210> 85
   <211> 50
   <212> RNA
   <213> artificial sequence
<220>
   <223> ARN en épingle à cheveu
<400> 85
   ugaagacuac auccaggacu ucaagagaag uccuggaugu agucuucauu 50
<210> 86
   <211> 21
   <212> DNA
   <213> artificial sequence
<220>
   <223> brin sens siRNA
<400> 86
   ugaagacuac auccaggacu u 21
<210> 87
   <211> 21
   <212> DNA
   <213> artificial sequence
<220>
   <223> brin antisens siRNA
<400> 87
   guccuggaug uagucuucau u 21

## Description

L'invention concerne des petits ARN interférents (*small interfering RNA* ou *silencing inducing RNA*), dénommés ci-après siRNA, spécifiques des sous-unités α, α' et β de la protéine kinase CK2 (ou caséine kinase 2) et leurs applications, notamment pour le traitement des cancers.

La protéine CK2 (ou caséine kinase 2) est une sérine/thréonine kinase pléiotrope et ubiquitaire, très conservée chez les eucaryotes ; cette holoenzyme est composée de deux sous-unités catalytiques α et α' et de deux sous-unités régulatrices β identiques associées en hétérotétramères αα'β₂, α'₂β₂ ou α₂β₂.

Cette protéine joue un rôle essentiel dans le contrôle de nombreux processus physiopathologiques ; elle est indispensable au développement embryonnaire et à la différenciation terminale, ainsi qu'au contrôle de la progression du cycle cellulaire et de la survie cellulaire et son expression est dérégulée dans de nombreux cancers incluant des tumeurs d'origine virale où elle participe au blocage de l'apoptose (Buchou et al., Mol. Cell. Biol., 2003, 23, 908-915 ; Ahmed et al., Trends in Cell Biology, 2002, 12, 226-230).

Du fait de son rôle essentiel dans de nombreux processus physiologiques et de l'importance des pathologies associées à son dysfonctionnement, la protéine CK2 représente une nouvelle cible pharmacologique pour le développement de médicaments, notamment des anti-cancéreux et des anti-viraux.

Toutefois, étant donné que l'invalidation des gènes des sous-unités de la CK2 est létale chez les souris transgéniques « knock-out » et non compatible avec la viabilité cellulaire (Buchou et al., précité), le développement de telles molécules est resté très limité en l'absence de modèle *in vivo* ou *in vitro* permettant l'analyse fonctionnelle du rôle des sous-unités de la CK2.

En effet, les quelques molécules capables d'inhiber la CK2 qui ont été décrites présentent l'inconvénient d'être, soit peu spécifiques, soit peu actives, à savoir :
- des petites molécules analogues de l'ATP capables d'inhiber spécifiquement les sous-unités catalytiques α et α' ; on peut citer comme analogue de l'ATP, le TBB (Sarno et al., FEBS lett., 2001, 496, 44-48), qui est un dérivé de DRB pour augmenter sa spécificité pour la sous-unité alpha de la CK2. Toutefois, ces analogues de substrats de kinases (ATP) peuvent inhiber l'activité d'autres protéines connues ou inconnues, mettant en oeuvre l'ATP cellulaire. La spécificité de tels produits et notamment du TBB étant incertaine, leur utilisation est exclue *in vivo,*
- des oligonucléotides anti-sens dirigés contre les sous-unités de la CK2 (Demande américaine US 2002/147163 et Brevet américain US 6 455 307 au nom de ISIS PHARMACEUTICALS INC ; Ulloa et al., EMBO, 1993, 12, 1633-1640 ; Faust et al., Head & Neck, 2000, 22, 341-346) ; l'inhibition de l'activité CK2, démontrée uniquement *in vitro,* est partielle, transitoire et nécessite des doses très élevées d'oligonucléotides anti-sens (plusieurs dizaines à plusieurs centaines de µg/ml en fonction de la sensibilité des cellules).

Il ressort de ce qui précède qu'il n'existe pas de molécules capables d'inhiber spécifiquement la protéine kinase CK2 de façon efficace. En outre, il n'existe pas de modèle *in vitro* ou *in vivo* permettant une analyse fonctionnelle de chacune des sous-unités CK2, utile pour le criblage de molécules capables de moduler l'activité de la protéine kinase CK2.

Il a été montré que des fragments d'ARN double-brin complémentaires d'un ARNm sont capables, lorsqu'ils sont introduits dans des cellules eucaryotes, d'inhiber fortement l'expression du gène correspondant en détruisant cet ARNm. Ce phénomène dénommé interférence ARN (pour une revue voir : Biofutur, 2002, volume 228, pages 52-61 ; Voorhoeve et al., TIBS, 2003, 21, 2-4 ; S. Brantl, Biochimica et Biophysica Acta, 2002, 1575, 15-25) a été mis en évidence et particulièrement bien étudié chez les plantes et les invertébrés (*Caenorhabditis elegans* (Parrish et al., Molecular Cell, 2000, 6, 1077-1087), drosophile (Elbashir et al., The EMBO Journal, 2001, 20, 6877-6888)) et l'on peut raisonnablement supposer l'existence d'un mécanisme semblable chez les animaux supérieurs, puisque l'interférence ARN a également été observée dans des cellules humaines en culture. Toutefois, il a été montré que chez les invertébrés le phénomène était même capable de se propager à l'ensemble de l'organisme et de persister après la division cellulaire, ce qui n'a pas été observé chez les animaux supérieurs.

Des petits fragments d'ARN double-brin, longs de 21 à 25 nucléotides, sont les véritables déclencheurs de l'inhibition. Ces siRNA peuvent pénétrer directement dans les cellules végétales et vraisemblablement aussi dans celles d'invertébrés. Chez la drosophile, il a été montré que les siRNA s'intègrent dans des complexes moléculaires appelés RISC (*RNA-induced silencing complex*). Avec l'intervention d'une hélicase et d'ATP comme source d'énergie, ces complexes exposent les brins du siRNA. Si la séquence génétique du siRNA correspond à un fragment d'un gène qui s'exprime naturellement dans la cellule, l'ARN interférant exposé par le complexe RISC va rencontrer un ARN messager porteur d'une séquence exactement complémentaire et les deux molécules vont s'associer. La présence du brin de siRNA provoque l'intervention d'enzymes qui vont sectionner l'ARN messager à l'endroit où il est lié au siRNA. Les deux parties de l'ARN messager sectionné, privées de l'une de leurs terminaisons habituelles, sont identifiées comme incomplètes et détruites par la cellule. L'ARN messager ciblé ne peut plus jouer son rôle et commander la synthèse d'une protéine. C'est ainsi que s'explique le caractère extrêmement spécifique de l'interférence ARN (R. Agami, Current Opinion in Chemical Biology, 2002, 6, 829-834 ; il n'y a réaction que si la séquence d'une vingtaine de nucléotides du siRNA a son homologue exact sur un ARN messager. La probabilité qu'un segment d'ADN, pris au hasard, corresponde à un siRNA donné est de l'ordre de 1/4 à la puissance 21 (21 nucléotides pouvant prendre chacun 4 «valeurs»), soit une chance sur plus de 4 milliards.

Ce phénomène d'inhibition spécifique de l'expression des gènes, ouvre des perspectives intéressantes dans le domaine de la génomique fonctionnelle et de la recherche pharmaceutique, respectivement pour identifier rapidement la fonction de nouveaux gènes, et pour sélectionner rapidement les gènes cibles et les médicaments candidats.

Ainsi, des siRNA spécifiques des ADNc codant pour des protéines virales ou cellulaires et capables d'inhiber la production des protéines correspondantes ont été décrits (p24 de HIV, gD de HSV, IL-12 (Demande Internationale PCT WO 00/63364) ; bcl-2 (Read et al., Expert. Opin. Ther. Patents, 2003, 13, 627-638)).

Toutefois, aucun siRNA capable d'inhiber spécifiquement l'expression des sous-unités de la protéine kinase CK2 n'a été décrit.

De manière surprenante, les Inventeurs ont isolé des siRNA spécifiques des transcrits des sous-unités α, α' et β de la CK2 capables de bloquer sélectivement l'expression de la sous-unité α, de la sous-unité α' ou de la sous-unité β de la protéine kinase CK2 dans des cellules, et ce de façon efficace, spécifique et durable. De tels siRNA qui présentent un effet inhibiteur prolongé, de l'ordre de 72 heures, à des concentrations faibles (de l'ordre de 20 nM, *in vitro),* sont utiles comme médicament pour le traitement des cancers. En effet, une concentration de 20 nM de siRNA inhibe plus de 80% de l'expression des sous-unités de la protéine kinase CK2 (détectée par Western Blot) et des ARNm correspondants (quantification par RT-PCR au light-cycler) après 48 h dans des cellules humaines (MCF7, HeLa ou fibroblastes 3T3).

En outre, ces ARN qui inhibent spécifiquement l'expression de la sous-unité α, α' ou β de la protéine kinase CK2 représentent également des outils pour l'analyse fonctionnelle du rôle respectif de chaque unité de la CK2 et le criblage de molécules capables de moduler (activer ou inhiber) l'activité d'une ou plusieurs de ces sous-unités de la CK2.

La présente invention a ainsi pour objet, un oligonucléotide double-brin formé de deux brins de 19 à 23 nucléotides, chaque brin étant constitué de 5' en 3' par une séquence de 17 à 21 ribonucléotides et deux désoxyribo- ou ribonucléotides, les séquences d'ARN de 17 à 21 ribonucléotides desdits brins étant complémentaires et les deux nucléotides des extrémités 3' étant protrusifs, caractérisé en ce que la séquence d'ARN du brin sens ou brin positif est celle d'un fragment d'un transcrit d'une sous-unité α, α' ou β d'une protéine kinase CK2 sélectionné dans le groupe constitué par :
a) un fragment correspondant à un oligonucléotide qui inhibe plus de 80 % de l'expression de la sous-unité correspondante, en culture cellulaire, à une concentration comprise entre 1 et 200 nM, de préférence inférieure à 20 nM,
b) un fragment d'un transcrit d'une sous-unité α inclus entre les positions 18-74, 259-279, 565-585, 644-664, 720-750, 808-831 et 863-885, à partir du codon ATG, en référence à la séquence d'ADNc de la sous-unité α de la CK2 de souris n° NM_007787 ou humaine n° NM_001895,
c) un fragment d'un transcrit d'une sous-unité α' inclus entre les positions 49-69, 132-152, 306-326, 367-387, 427-447, 451-471, 595-615, 735-755, 827-847, 868-888, 949-969 et 988-1008, à partir du codon ATG, en référence à la séquence d'ADNc de la sous-unité α' de la CK2 de souris NM_009974 ou humaine n° NM_001896,
d) un fragment d'un transcrit d'une sous-unité β inclus entre les positions 80-100, 116-137, 164-208, 369-389, 400-420, 527-591 et 613-643, à partir du codon ATG, en référence à la séquence d'ADNc de la sous-unité β de la CK2 humaine n° NM_001320 ou de souris n° NP_034105, et
e) un fragment de 17 à 21 nucléotides présentant au moins 80 % d'identité avec les fragments définis en a), b), c) et d).

L'oligonucléotide double-brin selon l'invention correspond à un siRNA capable d'inhiber l'expression de la sous-unité correspondante de la protéine kinase CK2 ; la séquence d'ARN de 17 à 21 nucléotides du brin sens ou brin positif est celle de la séquence-cible du transcrit de la sous-unité α, α' ou β de la protéine kinase CK2 de mammifère.

L'invention englobe les oligonucléotides naturels, synthétiques, semi-synthétiques ou recombinants ciblant la protéine kinase CK2 de n'importe qu'elle organisme, notamment eucaryote. Compte tenu des informations données en référence aux séquences humaines et de souris, l'Homme du métier est en mesure de trouver les positions équivalentes dans les séquences d'autres organismes eucaryotes, notamment de mammifères, accessibles dans les bases de données de séquences

Conformément à l'invention, l'identité d'une séquence oligonucléotidique par rapport à une séquence de référence s'apprécie en fonction du pourcentage de nucléotides qui sont identiques, lorsque les séquences sont alignées, de manière à obtenir le maximum de correspondance entre elles.

Selon un mode de réalisation avantageux dudit oligonucléotide double-brin, ladite séquence est sélectionnée dans le groupe constitué par :
a) un fragment d'une sous-unité α défini par l'équivalent ARN de la séquence SEQ ID NO: 1 à 13,
b) un fragment d'une sous-unité α' défini par l'équivalent ARN de la séquence SEQ ID NO: 14 à 25,
c) un fragment d'une sous-unité β défini par l'équivalent ARN de la séquence SEQ ID NO: 26 à 40, et
d) une séquence telle que définie en a), b) ou c), tronquée d'un ou deux nucléotides à son extrémité 5' et/ou 3', pourvu que la longueur de ladite séquence ne soit pas inférieure à 17 ribonucléotides.

Au sens de la présente invention, on entend par équivalent ARN d'une séquence d'ADN, la séquence dans laquelle les désoxyribonucléotides (a, g, c, t) de la dite séquence d'ADN sont remplacés par les ribonucléotides (a, g, c, u).

Selon un autre mode de réalisation avantageux dudit oligonucléotide double-brin, chacun des brins comprend un groupement phosphate en 5' et un groupement hydroxyle en 3'.

Selon encore un autre mode de réalisation avantageux dudit oligonucléotide double-brin, lesdits nucléotides protrusifs des extrémités 3' sont sélectionnés dans le groupe constitué par les paires tt et aa.

Selon encore un autre mode de réalisation avantageux dudit oligonucléotide double-brin, il est formé de deux brins de 19 ou 20 nucléotides.

Selon une disposition avantageuse de ce mode de réalisation dudit oligonucléotide double-brin, il comprend un brin sens défini par la séquence SEQ ID NO:67 ou 68.

Selon encore un autre mode de réalisation avantageux dudit oligonucléotide double-brin, il est formé de deux brins de 21 à 23 nucléotides.

Selon une disposition avantageuse de ce mode de réalisation dudit oligonucléotide double-brin, il comprend un brin sens défini par la séquence SEQ ID NO:41 à 66, 69 à 81, 83 et 86.

Les Tableaux I, II et III suivants résument les propriétés des différents oligonucléotides de séquences SEQ ID NO: 1 à 86.

**TABLEAU I : séquences cibles et SiRNA α**

| Nom et n° | séquence cible de souris (séquence sens) | SiRNA | Taille | Tm | %GC | Position/codon ATG NM_007787 souris et NM_001895 de la séquence humaine | Homologie Hu*/souris |
|---|---|---|---|---|---|---|---|
| CK2α1 | *cagaccccgagagtactggga* (SEQ ID NO:3) | | 21 | **61.5** | 57 | 54 | 2 |
| CK2α2 | aacacacacagaccccgagag (SEQ ID NO:2) | | 21 | **61.7** | 52.4 | 46 | 2 |
| CK2α3 | aagcagggccagagtttacac (SEQ ID NO:1) | | 21 | **58.6** | 52.4 | 18 | 0 |
| CK2α4 | aacacacacagaccccgagag (SEQ ID NO:2) | | 21 | **59.3** | 52.4 | 46 | 2 |
| CK2α5 | aatttgagaggtgggcccaac (SEQ ID NO:4) | | 21 | **59.8** | 52.4 | 259 | 2 |
| CK2α6 | aatgtccgagttgcttctcga (SEQ ID NO:5) | | 21 | **58.8** | 47.6 | 565 | 1 |
| CK2α7 | aacgatatcttgggcagacac (SEQ ID NO:10) | | 21 | **57.9** | 47.6 | 808 | 1 |
| CK2α8 | aaaaccagcatcttgtcagcc (SEQ ID NO:12) | | 21 | **60.3** | 47.6 | 863 | 2 |
| CK2α9 | aaccagcatcttgtcagccct (SEQ ID NO:13) | | 21 | **62.0** | 52.4 | 865 | 2 |
| CK2α10 | aggatagccaaggttctgg (SEQ ID NO:9) | | 21 | **58.9** | 47.6 | 730 | 0 |
| CK2α11 | tggtgaggatagccaaggttc (SEQ ID NO:8) | | 21 | **57.1** | 47.6 | 725 | 0 |
| CK2α12 | tcagttggtgaggatagcca (SEQ ID NO:7) | | 21 | **58.8** | 47.6 | 720 | 0 |
| CK2α13 | gatatcttgggcagacactcc (SEQ ID NO:11) | | 21 | **58.6** | 47.6 | 811 | 1 |
| CK2α14 | tgtggagcttgggttgtatgc (SEQ ID NO:6) | | 21 | **61.8** | 47.8 | 644 | 1 |

| | | | | | | | |
|---|---|---|---|---|---|---|---|
| * Hu = humain NB : l'ATG est en position 1 de la séquence de souris n° NM_007787 et en position 277 de la séquence humaine n° NM_001895. | | | | | | | |

**TABLEAU II : séquences cibles et SiRNA α'**

| Nom | Séquence cible humaine (séquence sens) | SiRNA | TAILLE | Tm | %GC | Position | Homologie Hu/souris |
|---|---|---|---|---|---|---|---|
| CK2α'1 | aacagtctgaggagccgcgag (SEQ ID NO:14) | | 21 | 66.5 | 66,7 | 49 | 1 mésappariement |
| CK2α,'2 | aaaacttggtcggggcaagta (SEQ ID NO:15) | | 21 | 59.5 | 47,6 | 132 | 2 mésappariements |
| CK2α'3 | aaaggaccctgtgtcaaagac (SEQ ID NO:16) | | 21 | 62.4 | 47,6 | 306 | 1 |
| CK2α'4 | aagcaactctaccagatcctg (SEQ ID NO:17) | | 21 | 55.8 | 47,6 | 367 | 0 |
| CK2α'5 | aaagctctggattactgccac (SEQ ID NO:18) | | 21 | 58.2 | 47,6 | 427 | 0 |
| CK2α'6 | aagggaatcatgcacagggat (SEQ ID NO:19) | | 21 | 62.8 | 47,6 | 451 | 0 |
| CK2α'7 | aagggaccagagctccttgtg (SEQ ID NO:20) | | 21 | 65.2 | 57,1 | 595 | 1 |
| CK2α'8 | aattgccaaggttctggggac (SEQ ID NO:21) | | 21 | 61.5 | 52,4 | 735 | 2 mais aux extrémités |
| CK2α'9 | aacattcacggaagcgctggg (SEQ ID NO:22) | | 21 | 66.4 | 57,1 | 827 | 1 |
| CK2α'10 | aacaggcaccttgtcagcccg (SEQ ID NO:23) | | 21 | 61.0 | 61,9 | 868 | 2 dont un le dernier nt |
| CK2α'11 | aaagaggccatggagcaccc a (SEQ ID NO:24) | | 21 | 68.4 | 57,1 | 949 | 0 |
| CK2α'12 | aaggagcagtcccagccttgt (SEQ ID NO:25) | | 21 | 64.6 | 57,1 | 988 | 0 |

| | | | | | | | |
|---|---|---|---|---|---|---|---|
| NB : l'ATG est en position 99 de la séquence de souris n° NM_009974 et en position 164 de la séquence humaine n° NM_001896. | | | | | | | |

**TABLEAU III : séquences cibles et SiRNA β**

| Nom | Séquence cible humaine (séquence sens) | SiRNA | Taille | Tm | %GC | Position | Homologie hu/souris |
|---|---|---|---|---|---|---|---|
| CK2β1 | aagacaaccccaaccagagtg (SEQ IDNO:32) | | 21 | 61.2 | 52.4 | 188 | 0 m ésappariement |
| CK2β2 | tcaatgagcaggtccctcact (SEQ ID NO:27) | | 19 | 62 | 52.4 | 116 | 0 |
| CK2β3 | acctggagcctgatgaagaac (SEQ ID NO:29) | | 21 | 60.5 | 52.4 | 164 | 1 |
| CK2β4 | tggagcctgatgaagaactgg (SEQ ID NO:30) | | 21 | 62.5 | 52.3 | 167 | 1 |
| CK2β5 | ggagcctgatgaagaactgga (SEQ ID NO:31) | | 21 | 62.5 | 52.3 | 168 | 1 |
| CK2β6 | caatgagcaggtccctcacta (SEQ ID NO:28) | | 21 | 60.1 | 52.3 | 117* | 0 |
| CK2β7 | ccaagagacctgccaaccagt (SEQ ID NO:35) | | 21 | 62 | 47.6 | 527 | 1 |
| CK2β8 | cctgtcggacatcccaggtga (SEQ ID NO:33) | | 21 | 62.2 | 52.3 | 369 | 3 |
| CK2β9 | agcaacttcaagagcccagtc (SEQ ID NO:38) | | 21 | 60.8 | 52.3 | 613 | 0 |
| CK2β10 | ccaggctctacggtttcaaga (SEQ ID NO:36) | | 21 | 60.5 | 52.3 | 554 | 1 |
| CK2β11 | agagcccagtcaagacgattc (SEQ ID NO:40) | | 21 | 60.6 | 52.3 | 623 | 0 |
| CK2β12 | aacttcaagagcccagtcaag (SEQ ID NO:39) | | 21 | 60.8 | 52.3 | 616 | 0 |
| CK2β13 | aagctctactgccccaagtgc (SEQ ID NO:34) | | 21 | 63 | 52.4 | 400 | 1 |
| CK2β14 | aagatccatccgatggcctac (SEQ ID NO:37) | | 21 | 62.3 | 42.9 | 571 | 2 |
| CK2β15 | aagactacatccaggacaat (SEQ ID NO:26) | | 20 | 52.1 | 38.1 | 80 | 0 |
| CK2β16 | aagactacatccaggacaat (SEQ ID NO:26) | | 21 | | | | |
| CK2β17 | aagactacatccaggacaat (SEQ ID NO:26) | | 21 | | | | |

| | | | | | | | |
|---|---|---|---|---|---|---|---|
| NB : l'ATG est en position 341 de la séquence humaine n° NM_001320. | | | | | | | |

Selon un mode de réalisation avantageux de l'oligonucléotide double-brin tel que défini ci-dessus, il est stabilisé.

Les oligonucléotides stabilisés sont connus de l'Homme du métier ; ils peuvent être stabilisés, notamment par incorporation de bases modifiées au cours de la synthèse *in vitro* ou par modifications des bases préalablement incorporées dans lesdits oligonucléotides. Des exemples de ces modifications sont présentés au Tableau IV.

La présente invention a également pour objet un précurseur de l'oligonucléotide double-brin tel que défini ci-dessus, caractérisé en ce qu'il est sélectionné dans le groupe constitué par :
a) un oligonucléotide simple-brin correspondant au brin sens ou antisens tel que défini ci-dessus,
b) un oligodésoxynucléotide (ADN) double-brin correspondant aux brins sens et/ou antisens de l'oligonucléotide double-brin tel que défini ci-dessus,
c) un oligoribonucléotide en épingle à cheveu comprenant les séquences des brins d'ARN sens et anti-sens telles que définies ci-dessus, et
d) un ADN double-brin formé d'un brin sens correspondant à l'équivalent ADN de l'oligoribonucléotide défini en c) et d'un brin antisens complémentaire du précédent.

Au sens de la présente invention, on entend par équivalent ADN d'une séquence d'ARN, la séquence dans laquelle les ribonucléotides (a, g, c, u) de la dite séquence d'ADN sont remplacés par les désoxyribonucléotides (a, g, c, t).

Les précurseurs sont utiles pour produire les oligonucléotides simple-brin et double-brin selon la présente invention par les techniques classiques de synthèse oligonucléotidique ou de transcription à l'aide d'un vecteur recombinant.

Chacun des brins du siRNA peut-être synthétisé séparément puis les brins complémentaires sont hybridés de façon à former des duplexes d'ARN. Alternativement, les brins du siRNA peuvent être synthétisés simultanément.

Le siRNA peut également être produit sous forme d'une molécule d'ARN en épingle à cheveu selon le principe décrit dans Brummelkamp et al., Science, 2002, 296, 550-553. La molécule d'ARN en épingle à cheveu est ensuite clivée dans les cellules transfectées par ladite molécule d'ARN ou transduites par un vecteur de transcription approprié, pour libérer le siRNA. Cette molécule d'ARN en épingle à cheveu comprend les séquences des deux brins du siRNA séparées par une courte séquence d'oligoribonucléotides non-complémentaires d'environ 3 à 12 nucléotides formant une boucle d'environ 5 à 15 nucléotides. Par exemple, une boucle d'environ 10 nucléotides est formée à partit d'une courte séquence d'environ 8 ribonucléotides et de deux nucléotides issus de l'extrémité 3' du brin sens du siRNA.

Les oligonucléotides simple-brin et double-brin selon la présente invention peuvent être, soit produits par synthèse chimique ou par transcription *in vitro* (tube à essai) ou en culture cellulaire, puis administrés *in vivo,* soit ils sont produits *in vivo* dans les cellules d'un organisme qui ont été modifiées par un vecteur de transcription (thérapie génique) ou un ADN codant lesdits siRNA (transgénèse).

La synthèse chimique est réalisée selon la méthode classique des phosphoramidites, décrite notamment dans Elbashir et al., Nature, 2001, 411, 494-498). Par exemple, chacun des brins du siRNA peut-être synthétisé selon chimie β-cyanoéthyl phosphoramidite sur support solide utilisant le 2'-O-tert-butyldimethylsilyl (TBDMS) comme groupement protecteur de la position 2' du ribonucléotide. D'autres groupements protecteur peuvent être utilisés ; le silyl ether qui protège l'extrémité 5'-hydroxyl du ribonucléotide peut-être utilisé en combinaison avec un orthoester labile qui protège le 2'-hydroxyl du ribonucléotide.

La transcription à l'aide d'un vecteur recombinant met en oeuvre un ADN double-brin codant pour l'un au moins ou les deux brins du siRNA ou bien un ARN en épingle à cheveu tel que défini ci-dessus. De tels ADN clonés dans des vecteurs d'expression appropriés, permettent la transcription séparée ou simultanée des deux brins complémentaires dudit siRNA, comme décrit respectivement dans Sadher et al. Biochem. Int., 1987 : 14, 1015 et dans le brevet européen EP 0618 966 au nom de CIS BIO INTERNATIONAL. Par exemple, le procédé de préparation d'ARN double-brin décrit dans le brevet européen EP 0618 966 met en oeuvre une matrice d'ADN fixée sur un support qui permet la transcription simultanée des deux brins d'ARN en ARN double-brin après une étape d'amplification (PCR) de la séquence ADN cible. L'ARN double-brin obtenu peut être fixé sur un support et plusieurs séquences différentes de siRNA peuvent être analysées simultanément.

L'invention a également pour objet une cassette d'expression, caractérisée en ce qu'elle comprend au moins un précurseur tel que défini ci-dessus sous le contrôle d'éléments régulateurs de la transcription appropriés, notamment un promoteur, inductible ou non et un terminateur de transcription.

L'invention a également pour objet un vecteur eucaryote ou procaryote comprenant un insert constitué par un oligonucléotide tel que défini ci-dessus ; de préférence ledit vecteur est un vecteur d'expression dans lequel est inséré une cassette d'expression telle que définie ci-dessus.

Ces vecteurs sont construits et introduits dans des cellules hôtes par les méthodes classiques d'ADN recombinant et de génie génétique, qui sont connues en elles-mêmes. De nombreux vecteurs dans lesquels on peut insérer une molécule d'acide nucléique d'intérêt afin de l'introduire et de la maintenir dans une cellule hôte eucaryote ou procaryote, sont connus en eux-mêmes ; le choix d'un vecteur approprié dépend de l'utilisation envisagée pour ce vecteur (par exemple réplication de la séquence d'intérêt, expression de cette séquence, maintien de la séquence sous forme extrachromosomique ou bien intégration dans le matériel chromosomique de l'hôte), ainsi que de la nature de la cellule hôte. On peut utiliser entre autres des vecteurs viraux tels que les adénovirus, les rétrovirus, les lentivirus et les AAV dans lesquels a été insérée préalablement la séquence d'intérêt ou bien des vecteurs non-viraux tels que des plasmides.

De manière préférée, ledit vecteur est un vecteur à ADN (plasmide ou virus recombinant) comprenant un oligodésoxynucléotide double-brin tel que défini ci-dessus ; un tel vecteur codant un siRNA tel que défini ci-dessus est utile pour la production *in vitro* ou *in vivo* desdits siRNA.

Des vecteurs particulièrement bien adaptés à l'expression stable des siRNA, sont notamment ceux décrits dans T.R. Brummelkamp et al., Science, 2002, 296, 550-553.

La présente invention a également pour objet des cellules eucaryotes ou procaryotes modifiées par un oligonucléotide, un précurseur, une cassette d'expression ou un vecteur tel que défini ci-dessus.

La présente invention a également pour objet un animal non-humain transgénique, caractérisé en ce qu'il comprend des cellules modifiées par un précurseur, une cassette d'expression ou un vecteur tel que défini ci-dessus.

La présente invention a également pour objet une composition pharmaceutique, caractérisée en ce qu'elle comprend au moins un oligonucléotide, un précurseur ou un vecteur codant ledit siRNA tels que définis ci-dessus et un véhicule pharmaceutiquement acceptable.

Lesdits oligonucléotides (oligonucléotide double-brin (siRNA), ou simple-brin ou précurseur des précédents), isolés ou insérés dans un vecteur tel que défini ci-dessus, sont introduits dans des cellules-cibles soit par diffusion passive, soit en utilisant des méthodes physiques telles que l'électroporation ou la microinjection, soit en les associant à toute(s) substance(s) permettant le passage de la membrane plasmique, tels que des transporteurs comme les nanotransporteurs, des liposomes, des lipides ou des polymères cationiques, tel que le phosphate de calcium (kit Sigma ref. CA-PHOS), l'amine (kit Ambion réf. 4502), la lipofectamine (kit Polyplus-transfection, réf. 101-05) ou le fugène-6 (Roche, réf 1815-091). En outre, on peut avantageusement combiner ces méthodes, par exemple en utilisant l'électroporation associée à des liposomes.

Dans certains cas, il n'est pas nécessaire d'associer les oligonucléotides selon l'invention avec une substance permettant leur passage à travers la membrane plasmique, dans la mesure où les siRNA sont assez petits pour diffuser librement dans les différents compartiments cellulaires. Ils peuvent agir au niveau du cytoplasme mais aussi à la membrane nucléaire, voire dans le noyau.

Selon un mode de réalisation avantageux de ladite composition, ledit oligonucléotide, ledit précurseur ou ledit vecteur sont associés à au moins une substance permettant le passage de la membrane plasmique tels que des transporteurs comme les nanotransporteurs, des liposomes, des lipides ou des polymères cationiques.

Selon un autre mode de réalisation avantageux de ladite composition, ledit oligonucléotide, ledit précurseur ou ledit vecteur sont associés à au moins une substance permettant le ciblage dans des cellules, des tissus ou des organes spécifiques tels que des anticorps et des peptides, notamment des peptides aptes à passer la barrière hématoencéphalique comme les peptides Pep:Trans^{™} (http://www.syntem.com/english/techpeptrans.html). D'autres peptides peuvent avantageusement être utilisés pour faciliter la transfection de siRNA au travers de la membrane plasmique des cellules ; les anticorps décrits dans Lu Z.R. et al. (Nature Biotechnol., 1999, 17, 1101-1104) peuvent notamment être utilisés pour cibler des cellules cancéreuses.

Selon encore un autre mode de réalisation avantageux de ladite composition, ledit oligonucléotide, ledit précurseur ou ledit vecteur sont associés à au moins un agent anti-viral ou anti-cancéreux.

Selon un autre mode de réalisation avantageux de ladite composition, elle comprend un mélange de plusieurs oligonucléotides ou de leurs précurseurs, ou bien un ou plusieurs vecteurs d'expression dudit mélange d'oligonucléotides, et notamment un mélange comprenant au moins un oligonucléotide spécifique de la sous-unité α, au moins un oligonucléotide spécifique de la sous-unité α' et au moins un oligonucléotide spécifique de la sous-unité β.

La présente invention a également pour objet l'utilisation d'un oligonucléotide, d'un précurseur ou d'un vecteur tels que définis ci-dessus pour la préparation d'un médicament destiné à la prévention et/ou au traitement du cancer.

La présente invention a également pour objet l'utilisation d'un oligonucléotide, d'un précurseur ou d'un vecteur tels que définis ci-dessus pour la préparation d'un médicament destiné à la prévention et/ou au traitement des maladies virales.

La présente invention a également pour objet un produit contenant au moins un oligonucléotide, un précurseur ou un vecteur tels que définis ci-dessus et un principe actif anti-cancéreux, en tant que préparation combinée pour une utilisation simultanée, séparée ou étalée dans le temps, dans la prévention et/ou le traitement du cancer.

La présente invention a également pour objet un produit contenant au moins un oligonucléotide, un précurseur ou un vecteur tels que définis ci-dessus et un principe actif anti-viral, en tant que préparation combinée pour une utilisation simultanée, séparée ou étalée dans le temps, dans la prévention et/ou le traitement des maladies virales.

La posologie utile varie en fonction de l'affection à traiter, de la voie et du rythme d'administration, ainsi que de la nature et du poids de l'espèce à traiter (humaine ou animale). Les oligonucléotides sont utilisés par voie digestive (orale, sublinguale), parentérale ou locale. Ils peuvent se présenter sous la forme de comprimés, simples ou dragéifiés, de gélules, de granules, de sirop, de suppositoires, de préparations injectables, de pommades, de crèmes, de gels, d'aérosol, lesquels sont préparés selon les méthodes usuelles. Dans ces formes galéniques, les oligonucléotides sont incorporés à des excipients habituellement employés dans des compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

*In vitro,* les concentrations qui peuvent être utilisées chez le rat sont comprises entre 10 nM et 200 µM ; les doses *in vivo* peuvent donc être comprises entre 1 µg et 20 mg/kg. Les doses correspondantes chez l'homme peuvent être déduites de ces informations.

L'invention a également pour objet l'utilisation d'un oligonucléotide, d'un précurseur, d'un vecteur, des cellules eucaryotes ou procaryotes modifiées ou d'un animal transgénique, tels que définis ci-dessus pour le criblage de molécules capables de moduler sélectivement l'activité des sous-unités α, α' ou β de la protéine CK2 ; par exemple, il est possible d'inhiber spécifiquement l'expression d'une des sous-unités *in vivo* ou *in vitro* et ainsi de cribler des molécules actives sur l'autre sous-unité ; de telles molécules représentent des médicaments potentiels, utiles pour la prévention et le traitement des pathologies liées à une dérégulation (augmentation ou diminution) de l'activité de la protéine kinase CK2 dans les cellules.

On peut citer, à titre d'exemple de pathologie liée à une dérégulation de l'activité de la CK2, l'infertilité masculine en l'absence de CK2 α' non compensée par α qui est absente dans les cellules germinales au dernier stade de différenciation des spermatogonies (Xu et al., Nature Gen., 1999, 23, 118-121).

Par rapport aux oligonucléotides anti-sens de l'art antérieur, les oligonucléotides et notamment les siRNA selon l'invention présentent les avantages suivants :
- ils sont stables *in vitro* et *in vivo,*
- ils sont actifs à des concentrations faibles (de l'ordre de 20 nM *in vitro*) et inhibent de façon très efficace (> 80 % d'inhibition) l'expression et par conséquent l'activité de la protéine kinase CK2 dans les cellules,
- ils présentent un effet prolongé, jusqu'à 6 jours.

Outre les dispositions qui précèdent, l'invention comprend encore d'autres dispositions qui ressortiront de la description qui va suivre, qui se réfère à des exemples de mise en oeuvre du siRNA correspondant à la séquence SEQ ID NO: 26 selon la présente invention ainsi qu'aux dessins annexés dans lesquels :

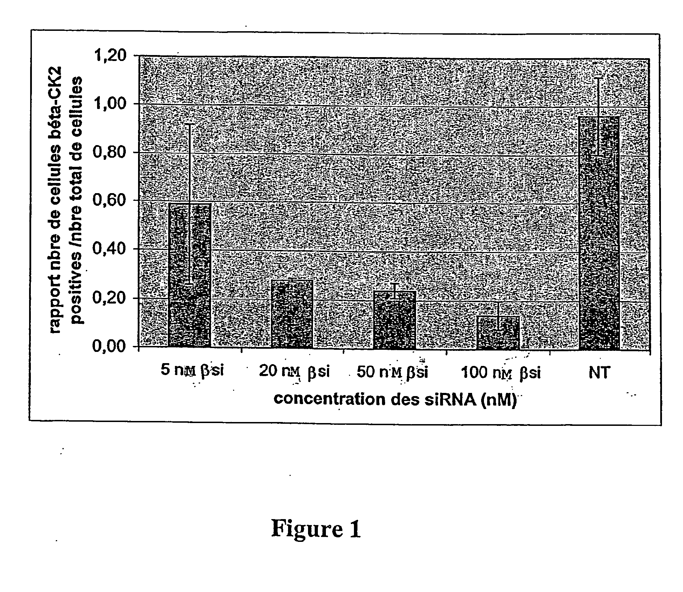

- la figure 1 illustre l'analyse par immunofluorescence de l'inhibition de l'expression de la sous-unité β de la protéine kinase CK2, par un siRNA ciblant la séquence SEQ ID NO:26 (βsi) ; les cellules ont été marquées avec un anticorps primaire anti-CK2β, un anticorps secondaire couplé à la fluorescéine (fluorescence verte), puis contre-colorées à l'iodure de propidium (fluorescence rouge). L'inhibition est mesurée par le rapport du nombre de cellules CK2β positives (fluorescence verte) sur le nombre total de cellules (fluorescence rouge). Les valeurs représentent les moyennes de deux mesures indépendantes ± SEM. Une concentration finale de 100 nM de siRNA inhibe 90 % de l'expression de la sous-unité β de la protéine kinase CK2.

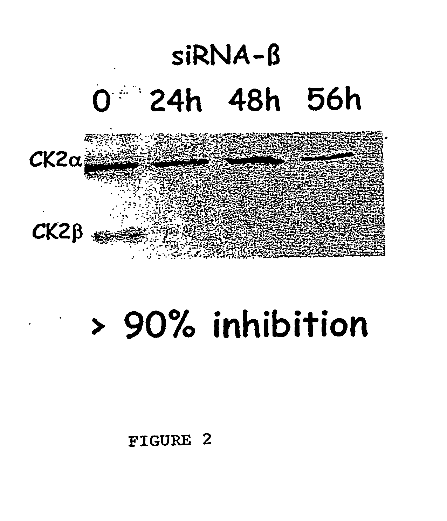

- la figure 2 illustre la cinétique d'inhibition de l'expression de la sous-unité β de la protéine kinase CK2 en présence d'un siRNA synthétique (siRNA-β) ciblant la séquence SEQ ID NO:26.

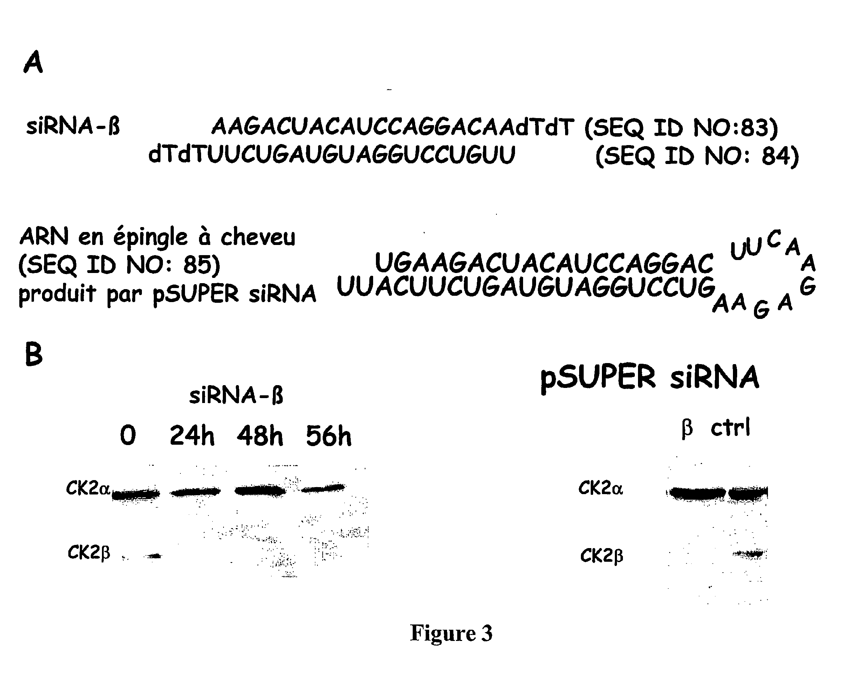

- la figure 3 illustre l'analyse comparative de l'inhibition de l'expression de la sous-unité β de la protéine kinase CK2 par un siRNA CK2β synthétique (séquence sens SEQ ID NO: 83 et antisens SEQ ID NO: 84) ou exprimé dans les cellules-cibles par un vecteur recombinant (pSUPERsiRNA) produisant un ARN en épingle à cheveu (SEQ ID NO: 85) correspondant au siRNA de séquence sens SEQ ID NO: 86 et antisens SEQ ID NO: 87,

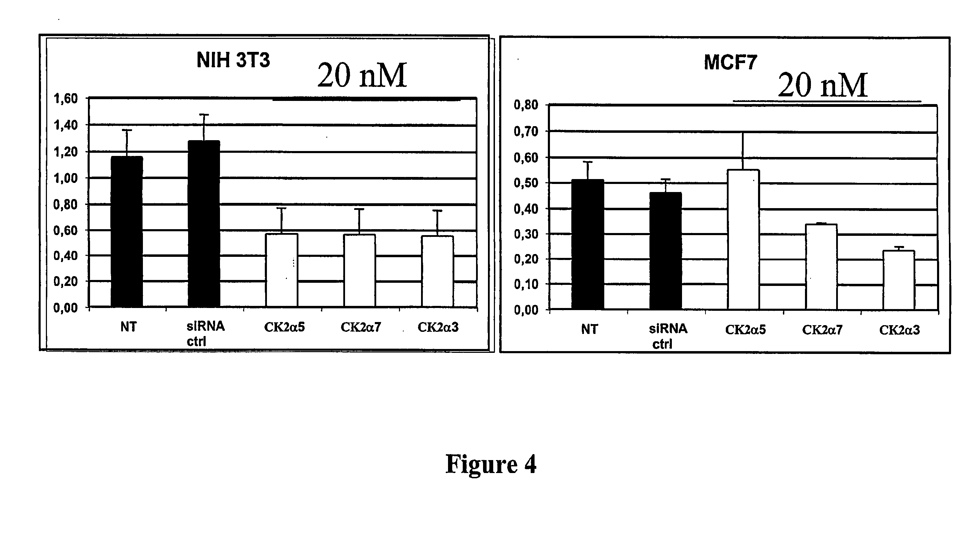

- la figure 4 illustre l'amélioration des séquences siRNA. Des cellules humaines (lignée MCF7) et murines (lignée NIH 3T3) ont été transfectées avec différents siRNA ciblant la sous-unité α (CK2α3, CK2α7, CK2α5) et présentant ou non des misappariements avec l'ARN-cible, et un siRNA contrôle (ctrl)). Les cellules ont été marquées avec un anticorps primaire anti-CK2α, un anticorps secondaire couplé à la fluorescéine (fluorescence verte), puis contre-colorées à l'iodure de propidium (fluorescence rouge). L'inhibition de l'expression de la sous-unité α est mesurée par le rapport du nombre de cellules CK2α positives (fluorescence verte) sur le nombre total de cellules (fluorescence rouge).

### EXEMPLE 1 : Expression d'un oligoribonucléotide selon l'invention dans les cellules-cibles modifiées par un vecteur recombinant

a) Une séquence d'ADN (gatcccctgaagactacatccaggacttcaagagagtcct ggatgtagtcttcatttttggaaa, SEQ ID NO:82) a été clonée dans le vecteur pSUPER selon les conditions décrites dans Brummelkamp TR et al. (Science, 2002, 296, 5567, 550-3). Le vecteur recombinant ainsi obtenu (pSUPER siRNA) permet l'expression d'un siRNA. ou CK2β ciblant la séquence SEQ ID NO:26, à partir d'un transcrit en épingle à cheveux (figure 3),
b) Des cellules NIH 3T3 sont transfectées avec les vecteurs obtenus en a) selon un protocole de transfection à l'aide de Fugène 6 (Roche).

### EXEMPLE 2 : Préparation d'oligoribonucléotides synthétiques, éventuellement stabilisés.

a) Les deux brins d'ARN sont synthétisés selon des méthodes connues (méthode au phosphoramidine ARN, voir notamment Elbashir S.M. et al., Nature, 2001, 411, 494-498).
b) Pour les stabiliser, il est avantageux de les modifier en insérant des nucléotides modifiés dans les deux brins d'ARN, au cours de la synthèse *in vitro.*

Le Tableau IV ci-après illustre des exemples de nucléotides modifiés.

| **Nucléotide modifié** | **Première Application** | **Seconde Application** |
|---|---|---|
| 2' F-CTP | Résistance à la nucléase | |
| 2' F-UTP | Résistance à la nucléase | |
| 2' NH₂-CTP | Résistance à la nucléase | |
| 2' NH₂-UTP | Résistance à la nucléase | |
| 2' N₃-CTP | Résistance à la nucléase | Modification post-synthèse |
| 2' N₃-UTP | Résistance à la nucléase | Modification post-synthèse |
| 2-thio CTP | Réticulation UV | |
| 2-thio UTP | Hybridation modifiée | Réticulation UV |
| 4-thio UTP | Hybridation modifiée | Réticulation UV |
| 5-iodo CTP | Réticulation UV | |
| 5-iodo UTP | Réticulation UV | |
| 5-bromo UTP | Réticulation UV | |
| 2-chloro ATP | Réticulation UV | |
| Adenosine 5'-(1-thiotriphosphate) | Instable chimiquement | résistance à la nucléase |
| Cytidine 5'-(1-thiotriphosphate) | Instable chimiquement | résistance à la nucléase |
| Guanosine -5'-(1-thiotriphosphate) | Instable chimiquement | résistance à la nucléase |
| Uridine -5'-(1-thiotriphosphate) | Instable chimiquement | résistance à la nucléase |
| Pseudo-UTP | | |
| 5-(3-aminoallyl)-UTP | Modification post-synthèse | |
| 5-(3-aminoallyl)-dUTP | Modification post-synthèse | |

De tels nucléotides sont notamment disponibles chez Ambion (http://www.ambion.com).

### EXEMPLE 3 : Inhibition de l'expression de la sous-unité β de la protéine kinase CK2 par un siRNA synthétique.

Des fibroblastes 3T3 ont été cultivés en goutte de 5 µl (2000 cellules) dans du milieu de culture complet, dans les puits d'une lame pour immuno-fluorescence (40 puits d'un diamètre de 2 mm ; lame en super téflon, référence 74890.01 (PROLABO)). Les cellules 3T3 ont été transfectées à l'aide du kit de transfection siPort® (AMBION), avec une concentration finale de 5, 20, 50 ou 100 nM de siRNA ciblant la séquence SEQ ID NO: 26, dans un volume de 5 µl, ou non-transfectées, puis les cellules ont été incubées pendant 2 jours à 37 °C. Les cellules ont ensuite été lavées et fixées à l'aide d'une solution de paraformaldéhyde (4 % dans PBS). Les cellules sont ensuite colorées à l'iodure de propidium et marquées à l'aide d'un anticorps primaire anti-sous-unité β de la protéine CK2 (anticorps βc ; Filhol et al 1994 Biochem Biophys Res Commun. 198 660-5) et d'un anticorps secondaire couplé à un fluorophore, tel que le cyanamide 3. La fluorescence est analysée à l'aide d'un scanner (Genomic Solution) et l'inhibition de l'expression de la protéine kinase CK2 est exprimée par le rapport du nombre de cellules exprimant la protéine kinase CK2 (cellules rouges marquées par l'anticorps βc) sur le nombre total de cellules (cellules bleues marquées à l'iodure de propidium).

Les résultats sont illustrés à la figure 1 et montrent qu'une concentration de 20 nM de siRNA inhibe 90 % de l'expression de la sous-unité β de la protéine kinase CK2.

### EXEMPLE 4 : Etude de l'inhibition de l'expression de la β CK2 par un siRNA synthétique ou produit dans les cellules-cibles modifiées par un vecteur d'expression

Les cellules NIH 3T3 transfectées, soit par un siRNA-β ciblant la séquence SEQ ID NO:26) (20 nM), soit par le vecteur d'expression correspondant (pSUPER siRNA), sont cultivées pendant les temps indiqués à la figure 2. Après lavage dans PBS, elles sont lysées dans du tampon TDG (Tris, HCl pH 7,4 10 mM, Glycérol 0,1 %, DTT 1 mM, NaCl 500 mM Triton X-100 0,1 %) et centrifugées 15 min à 15000g à 4°C. Le surnageant est dosé pour son contenu en protéines et 40 µg sont analysés par SDS-PAGE. Les protéines sont alors transférées sur membrane PVDF. Après saturation de la membrane dans du PBS contenant 0,05% Tween 20 et 3% de BSA pendant 1 heure, la sous-unité CK2β est révélée à l'aide de l'anticorps βC.

On observe une inhibition de l'expression de la sous-unité β de la protéine CK2 avec le siRNA synthétique ou produit par un vecteur d'expression (figure 3) ; cette inhibition est observée dès 24h comme le montre la cinétique d'inhibition avec le siRNA synthétique (figures 2 et 3).

### Exemple 5 : Amélioration des séquences siRNA

L'inhibition de l'expression de la sous-unité α de la protéine CK2 humaine ou murine par différents siRNA (CK2α3, CK2α7, CK2α5) présentant ou non des misappariements avec l'ARN-cible a été analysée par immunofluorescence comme décrit à l'exemple 3. Les siRNA sont spécifiques du transcrit murin et présentent 0 (CK2α3), 1 (CK2α7) ou 2 (CK2α5) misappariement(s) avec le transcrit humain (Tableau I) ; pour α7, le misappariement est dans la partie 5' de la séquence cible, alors que pour α5, le misappariement est dans la partie 5'de la séquence cible

Des cellules humaines (lignée MCF7) et murines (lignée NIH 3T3) ont été transfectées avec différents siRNA ciblant la sous-unité α (CK2α3, CK2α7, CK2α5) ou avec un siRNA contrôle (ctrl). Les cellules ont été marquées avec un anticorps primaire anti-CK2α, un anticorps secondaire couplé à la fluorescéine (fluorescence verte), puis contre-colorées à l'iodure de propidium (fluorescence rouge). L'inhibition de l'expression de la sous-unité α a été mesurée par le rapport du nombre de cellules CK2α positives (fluorescence verte) sur le nombre total de cellules (fluorescence rouge).

Les résultats montrent que la présence de misappariement avec l'ARN-cible diminue l'efficacité des siRNA (figure 4 ; voir α5 dans la lignée humaine par rapport à la lignée murine).

## Revendications

1. Oligonucléotide double-brin formé de deux brins de 19 à 23 nucléotides, chaque brin étant constitué de 5' en 3' par une séquence de 17 à 21 ribonucléotides et deux désoxyribo- ou ribonucléotides, les séquences d'ARN de 17 à 21 ribonucléotides desdits brins étant complémentaires et les deux nucléotides des extrémités 3' étant protrusifs, **caractérisé en ce que** la séquence d'ARN du brin sens ou brin positif est celle d'un fragment d'un transcrit d'une sous-unité α, α' ou β d'une protéine kinase CK2 sélectionné dans le groupe constitué par :
a) un fragment correspondant à un oligonucléotide qui inhibe plus de 80 % de l'expression de la sous-unité correspondante, en culture cellulaire, à une concentration comprise entre 1 et 200 nM, de préférence inférieure à 20 nM,
b) un fragment d'un transcrit d'une sous-unité α inclus entre les positions 18-74, 259-279, 565-585, 644-664, 720-750, 808-831 et 863-885, à partir du codon ATG, en référence à la séquence d'ADNc de la sous-unité α de la CK2 de souris n° NM_007787 ou humaine n° NM_001895,
c) un fragment d'un transcrit d'une sous-unité α' inclus entre les positions 49-69, 132-152, 306-326, 367-387, 427-447, 451-471, 595-615, 735-755, 827-847, 868-888, 949-969 et 988-1008, à partir du codon ATG, en référence à la séquence d'ADNc de la sous-unité α' de la CK2 de souris NM_009974 ou humaine n° NM_001896,
d) un fragment d'un transcrit d'une sous-unité β inclus entre les positions 80-100, 116-137, 164-208, 369-389, 400-420, 527-591 et 613-643, à partir du codon ATG, en référence à la séquence d'ADNc de la sous-unité β de la CK2 humaine n° NM_001320 ou de souris n° NP_034105, et
e) un fragment de 17 à 21 bases présentant au moins 80 % d'identité avec les fragments définis en a), b), c) et d).

2. Oligonucléotide double-brin selon la revendication 1, **caractérisé**
**en ce que** ladite séquence d'ARN de 17 à 21 ribonucléotides du brin sens est sélectionnée dans le groupe constitué par :
a) un fragment d'une sous-unité α défini par l'équivalent ARN de la séquence SEQ ID NO: 1 à 13,
b) un fragment d'une sous-unité α' défini par l'équivalent ARN de la séquence SEQ ID NO: 14 à 25,
c) un fragment d'une sous-unité β défini par l'équivalent ARN de la séquence SEQ ID NO: 26 à 40, et
d) une séquence telle que définie en a), b) ou c), tronquée d'un ou deux ribonucléotides à son extrémité 5'et/ou 3', pourvu que la longueur de ladite séquence ne soit pas inférieure à 17 nucléotides.

3. Oligonucléotide double-brin selon la revendication 1 ou la revendication 2, **caractérisé en ce que** chacun des brins comprend un groupement phosphate en 5' et un groupement hydroxyle en 3'.

4. Oligonucléotide double-brin selon l'une quelconque des revendications 1 à 3, **caractérisé en ce que** lesdits nucléotides protrusifs des extrémités 3' sont sélectionnés dans le groupe constitué par les paires tt et aa.

5. Oligonucléotide double-brin selon l'une quelconque des revendications 1 à 4, **caractérisé en ce qu'**il est formé de deux brins de 19 ou 20 nucléotides.

6. Oligonucléotide double-brin selon la revendication 5, **caractérisé en ce que** le brin sens est défini par la séquence SEQ ID NO:67 ou 68.

7. Oligonucléotide double-brin selon l'une quelconque des revendications 1 à 4, **caractérisé en ce qu'**il est formé de deux brins de 21 à 23 nucléotides.

8. Oligonucléotide double-brin selon la revendication 7, **caractérisé en ce que** le brin sens est défini par la séquence SEQ ID NO:41 à 66, 69 à 81, 83 et 86.

9. Oligonucléotide selon l'une quelconque des revendications 1 à 8, **caractérisé en ce qu'**il est stabilisé.

10. Précurseur de l'oligonucléotide selon l'une quelconque des revendications 1 à 9, **caractérisé en ce qu'**il est sélectionné dans le groupe constitué par :
a) un oligonucléotide simple-brin constitué par le brin sens ou antisens de l'oligonucléotide selon l'une quelconque des revendications 1 à 9,
b) un oligodésoxynucléotide double-brin correspondant aux brins sens et/ou antisens de l'oligonucléotide selon l'une quelconque des revendications 1 à 9,
c) un oligoribonucléotide en épingle à cheveu comprenant les séquences des brins sens et anti-sens de l'oligonucléotide double-brin selon l'une quelconque des revendications 1 à 9,
d) un oligodésoxynucléotide double-brin formé d'un brin sens correspondant à l'oligoribonucléotide défini en c) et d'un brin antisens complémentaire du précédent.

11. Cassette d'expression, **caractérisée en ce qu'**elle comprend au moins un précurseur tel que défini à la revendication 10, sous le contrôle d'éléments régulateurs de la transcription appropriés.

12. Vecteur d'expression, **caractérisé en ce qu'**il comprend la cassette telle que définie à la revendication 11.

13. Vecteur d'expression selon la revendication 12, **caractérisé en ce qu'**il s'agit d'un vecteur à ADN comprenant un précurseur ADN tel que défini en b) et d) de revendication 10 inclus dans une cassette d'expression.

14. Cellule eucaryote ou procaryote, **caractérisée en ce qu'**elle est modifiée par un oligonucléotide selon l'une quelconque des revendications 1 à 9, un précurseur selon la revendication 10, une cassette d'expression selon la revendication 11 ou un vecteur d'expression selon la revendication 12 ou la revendication 13.

15. Animal non-humain transgénique, **caractérisé en ce qu'**il comprend des cellules modifiées par un précurseur selon la revendication 10, une cassette d'expression selon la revendication 11 ou un vecteur d'expression selon la revendication 12 ou la revendication 13.

16. Composition pharmaceutique, **caractérisée en ce qu'**elle comprend au moins un oligonucléotide selon l'une quelconque des revendications 1 à 9, un précurseur selon la revendication 10 ou un vecteur d'expression selon la revendication 12 ou la revendication 13, et un véhicule pharmaceutiquement acceptable.

17. Composition pharmaceutique selon la revendication 16, **caractérisée en ce que** le dit oligonucléotide, précurseur ou vecteur sont associés à au moins une substance permettant le passage de la membrane plasmique.

18. Composition pharmaceutique selon la revendication 16 ou la revendication 17, **caractérisée en ce que** le dit oligonucléotide, précurseur ou vecteur sont associés à au moins une substance permettant le ciblage dans des cellules, des tissus ou des organes.

19. Composition pharmaceutique selon l'une quelconque des revendications 16 à 18, **caractérisée en ce que** le dit oligonucléotide, précurseur ou vecteur sont associés à au moins un agent anti-viral ou anti-cancéreux.

20. Composition pharmaceutique selon l'une quelconque des revendications 16 à 19, **caractérisée en ce qu'**elle comprend un mélange de plusieurs oligonucléotides ou de leurs précurseurs, ou bien un ou plusieurs vecteurs d'expression dudit mélange d'oligonucléotides, notamment un mélange comprenant au moins un oligonucléotide spécifique de la sous-unité oc, au moins un oligonucléotide spécifique de la sous-unité α' et au moins un oligonucléotide spécifique de la sous-unité β.

21. Utilisation d'un oligonucléotide selon l'une quelconque des revendications 1 à 9, d'un précurseur selon la revendication 10 ou d'un vecteur d'expression selon la revendication 12 ou la revendication 13, pour la préparation d'un médicament destiné à la prévention et/ou au traitement du cancer.

22. Utilisation d'un oligonucléotide selon l'une quelconque des revendications 1 à 9, d'un précurseur selon la revendication 10 ou d'un vecteur d'expression selon la revendication 12 ou la revendication 13, pour la préparation d'un médicament destiné à la prévention et/ou au traitement des maladies virales.

23. Produit contenant au moins un oligonucléotide selon l'une quelconque des revendications 1 à 9, un précurseur selon la revendication 10 ou un vecteur d'expression selon la revendication 12 ou la revendication 13 et un principe actif anti-cancéreux, en tant que préparation combinée pour une utilisation simultanée, séparée ou étalée dans le temps, dans la prévention et/ou le traitement du cancer.

24. Produit contenant au moins un oligoribonucléotide selon l'une quelconque des revendications 1 à 19, un précurseur selon la revendication 10 ou un vecteur d'expression selon la revendication 12 ou la revendication 13 et un principe actif anti-viral, en tant que préparation combinée pour une utilisation simultanée, séparée ou étalée dans le temps, dans la prévention et/ou le traitement des maladies virales.

25. Utilisation d'un oligoribonucléotide selon l'une quelconque des revendications 1 à 9, d'un précurseur selon la revendication 10, d'un vecteur d'expression selon la revendication 12 ou la revendication 13, de cellules eucaryotes ou procaryotes selon la revendication 14 ou d'un animal non-humain transgénique selon la revendication 15, pour le criblage de molécules capables de moduler l'activité des sous-unités α, α' ou β de la protéine kinase CK2.

## Patentansprüche

1. Doppelsträngiges Oligonucleotid, das aus zwei Strängen mit 19 bis 23 Nucleotiden gebildet ist, wobei jeder Strang von 5' nach 3' durch eine Sequenz aus 17 bis 21 Ribonucleotiden und zwei Desoxyribo- oder Ribonucleotiden gebildet wird, wobei die RNA-Sequenzen aus 17 bis 21 Ribonucleotiden der Stränge komplementär sind und die zwei Nucleotide der 3' -Enden vorstehend sind, dadurch **gekenn- zeichnet**, dass die RNA-Sequenz des Sense-Strangs oder positiven Strangs die eines Fragments eines Transkripts einer α-, α'- oder β-Untereinheit einer CK2-Proteinkinase ist, das ausgewählt ist aus der Gruppe, bestehend aus:
a) einem Fragment, das einem Oligonucleotid entspricht, das mehr als 80% der Expression der entsprechenden Untereinheit, in Zellkultur, bei einer Konzentration zwischen 1 und 200 nM, vorzugsweise von unter 20 nM, hemmt,
b) einem Fragment eines Transkripts einer α-Untereinheit, eingeschlossen zwischen den Positionen 18-74, 259-279, 565-585, 644-664, 720-750, 808-831 und 863-885, ausgehend von dem Codon ATG, in Bezug auf die cDNA-Sequenz der α-Untereinheit der CK2 der Maus Nr. NM_007787 oder der humanen Nr. NM_001895,
c) einem Fragment eines Transkripts einer α'-Untereinheit, eingeschlossen zwischen den Positionen 49-69, 132-152, 306-326, 367-387, 427-447, 451-471, 595-615, 735-755, 827-847, 868-888, 949-969 und 988-1008, ausgehend von dem Codon ATG, in Bezug auf die cDNA-Sequenz der α'-Untereinheit der CK2 der Maus NM_009974 oder der humanen Nr. NM_001896,
d) einem Fragment eines Transkripts einer β-Untereinheit, eingeschlossen zwischen den Positionen 80-100, 116-137, 164-208, 369-389, 400-420, 527-591 und 613-643, ausgehend von dem Codon ATG, in Bezug auf die cDNA-Sequenz der β-Untereinheit der humanen CK2 Nr. NM_001320 oder der CK2 der Maus Nr. NP_034105 und
e) einem Fragment aus 17 bis 21 Basen, das wenigstens 80% Identität mit den in a), b), c) und d) definierten Fragmenten aufweist.

2. Doppelsträngiges Oligonucleotid gemäß Anspruch 1, **dadurch gekennzeichnet, dass** die RNA-Sequenz aus 17 bis 21 Ribonucleotiden des Sense-Strangs ausgewählt ist aus der Gruppe, bestehend aus:
a) einem Fragment einer α-Untereinheit, das durch das RNA-Äquivalent der Sequenz SEQ ID NO:1 bis 13 definiert ist,
b) einem Fragment einer α'-Untereinheit, das durch das RNA-Äquivalent der Sequenz SEQ ID NO:14 bis 25 definiert ist,
c) einem Fragment einer β-Untereinheit, das durch das RNA-Äquivalent der Sequenz SEQ ID NO:26 bis 40 definiert ist, und
d) einer Sequenz, wie sie in a), b) oder c) definiert ist, die an ihrem 5'- und/oder 3'-Ende um ein oder zwei Ribonucleotide verkürzt ist, mit der Maßgabe, dass die Länge der Sequenz nicht unter 17 Nucleotide ist.

3. Doppelsträngiges Oligonucleotid gemäß Anspruch 1 oder Anspruch 2, **dadurch gekennzeichnet, dass** jeder der Stränge in 5' eine Phosphatgruppierung und in 3' eine Hydroxylgruppierung umfasst.

4. Doppelsträngiges Oligonucleotid gemäß einem der Ansprüche 1 bis 3, **dadurch gekennzeichnet dass** die vorstehenden Nucleotide der 3'-Enden aus der Gruppe, bestehend aus den Paaren tt und aa, ausgewählt sind.

5. Doppelsträngiges Oligonucleotid gemäß einem der Ansprüche 1 bis 4, **dadurch gekennzeichnet, dass** es aus zwei Strängen von 19 oder 20 Nucleotiden gebildet wird.

6. Doppelsträngiges Oligonucleotid gemäß Anspruch 5, **dadurch gekennzeichnet, dass** der Sense-Strang durch die Sequenz SEQ ID NO:67 oder 68 definiert wird.

7. Doppelsträngiges Oligonucleotid gemäß einem der Ansprüche 1 bis 4, **dadurch gekennzeichnet, dass** es aus zwei Strängen von 21 bis 23 Nucleotiden gebildet wird.

8. Doppelsträngiges Oligonucleotid gemäß Anspruch 7, **dadurch gekennzeichnet, dass** der Sense-Strang durch die Sequenz SEQ ID NO:41 bis 66, 69 bis 81, 83 und 86 definiert wird.

9. Oligonucleotid gemäß einem der Ansprüche 1 bis 8, dadurch **gekennzeeichnet**, dass es stabilisiert ist.

10. Vorläufer des Oligonuleotids gemäß einem der Ansprüche 1 bis 9, **dadurch gekennzeichnet, dass** es ausgewählt ist aus der Gruppe, bestehend aus:
a) einem einzelsträngigen Oligonucleotid, das durch den Sense- oder Antisense-Strang des Oligonucleotids gemäß einem der Ansprüche 1 bis 9 gebildet wird,
b) einem doppelsträngigen Oligodesoxynucleotid, das den Sense- und/oder Antisense-Strängen des Oligonucleotids gemäß einem der Ansprüche 1 bis 9 entspricht,
c) einem Haarnadel-Oligoribonucleotid, das die Sequenzen der Sense- und Antisense-Stränge des doppelsträngigen Oligonucleotids gemäß einem der Ansprüche 1 bis 9 umfasst,
d) einem doppelsträngigen Oligodesoxynucleotid, das aus einem Sense-Strang, entsprechend dem Oligoribonucleotid, das in c) definiert ist, und einem Antisense-Strang, der zu dem vorstehenden komplementär ist, gebildet wird.

11. Expressionskassette, **dadurch gekennzeichnet, dass** sie wenigstens einen Vorläufer, wie in Anspruch 10 definiert ist, unter der Kontrolle von geeigneten regulatorischen Elementen der Transkription umfasst.

12. Expressionsvektor, **dadurch gekennzeichnet, dass** er die Kassette, wie sie in Anspruch 11 definiert ist, umfasst.

13. Expressionsvektor gemäß Anspruch 12, **dadurch gekennzeichnet, dass** es sich um einen DNA-Vektor handelt, der einen DNA-Vorläufer, wie in b) und d) von Anspruch 10 definiert, der in einer Expressionskassette enthalten ist, umfasst.

14. Eukaryoten- oder Prokaryotenzelle, dadurch **gekenn**- **zeichnet**, dass sie durch ein Oligonucleotid gemäß einem der Ansprüche 1 bis 9, einen Vorläufer gemäß Anspruch 10, eine Expressionskassette gemäß Anspruch 11 oder einen Expressionsvektor gemäß Anspruch 12 oder Anspruch 13 modifiziert ist.

15. Nichtmenschliches transgenes Tier, **dadurch gekennzeichnet, dass** es Zellen umfasst, die durch einen Vorläufer gemäß Anspruch 10, eine Expressionskassette gemäß Anspruch 11 oder einen Expressionsvektor gemäß Anspruch 12 oder Anspruch 13 modifiziert sind.

16. Pharmazeutische Zusammensetzung, **dadurch gekennzeichnet, dass** sie wenigstens ein Oligonucleotid gemäß einem der Ansprüche 1 bis 9, einen Vorläufer gemäß Anspruch 10 oder einen Expressionsvektor gemäß Anspruch 12 oder Anspruch 13 und ein pharmazeutisch annehmbares Vehikel umfasst.

17. Pharmazeutische Zusammensetzung gemäß Anspruch 16, **dadurch gekennzeichnet, dass** das Oligonucleotid, der Vorläufer oder der Vektor mit wenigstens einer Substanz assoziiert sind, die die Passage durch die Plasmamembran erlaubt.

18. Pharmazeutische Zusammensetzung gemäß Anspruch 16 oder Anspruch 17, **dadurch gekennzeichnet, dass** das Oligonucleotid, der Vorläufer oder der Vektor mit wenigstens einer Substanz assoziiert sind, die die Targetierung in Zellen, Gewebe oder Organen ermöglicht.

19. Pharmazeutische Zusammensetzung gemäß einem der Ansprüche 16 bis 18, **dadurch gekennzeichnet, dass** das Oligonucleotid, der Vorläufer oder der Vektor mit wenigstens einem antiviralen oder Antikrebs-Mittel assoziiert sind.

20. Pharmazeutische Zusammensetzung gemäß einem der Ansprüche 16 bis 19, **dadurch gekennzeichnet, dass** sie ein Gemisch aus mehreren Oligonucleotiden oder ihren Vorläufern oder auch einem Expressionsvektor oder mehreren Expressionsvektoren des Oligonucleotidgemisches, insbesondere ein Gemisch, das wenigstens ein spezifisches Oligonucleotid der α-Untereinheit, wenigstens ein spezifisches Oligonucleotid der α'-Untereinheit und wenigstens ein spezifisches Oligonucleotid der β-untereinheit umfasst.

21. Verwendung eines Oligonucleotids gemäß einem der Ansprüche 1 bis 9, eines Vorläufers gemäß Anspruch 10 oder eines Expressionsvektors gemäß Anspruch 12 oder Anspruch 13 für die Herstellung eines Medikaments, das zur Prävention und/oder Behandlung von Krebs bestimmt ist.

22. Verwendung eines Oligonucleotids gemäß einem der Ansprüche 1 bis 9, eines Vorläufers gemäß Anspruch 10 oder eines Expressionsvektors gemäß Anspruch 12 oder Anspruch 13 für die Herstellung eines Medikaments, das zur Prävention und/oder Behandlung von Viruserkrankungen bestimmt ist.

23. Produkt, das wenigstens ein Oligonucleotid gemäß einem der Ansprüche 1 bis 9, einen Vorläufer gemäß Anspruch 10 oder einen Expressionsvektor gemäß Anspruch 12 oder 13 und einen Wirkstoff gegen Krebs enthält, als Kombinationspräparat für eine gleichzeitige, getrennte oder zeitlich ausgedehnte Verwendung bei der Prävention und/oder Behandlung von Krebs.

24. Produkt, das wenigstens ein Oligoribonucleotid gemäß einem der Ansprüche 1 bis 19, einen Vorläufer gemäß Anspruch 10 oder einen Expressionsvektor gemäß Anspruch 12 oder Anspruch 13 und einen antiviralen Wirkstoff enthält, als Kombinationspräparat für eine gleichzeitige, getrennte oder zeitlich ausgedehnte Verwendung bei der Prävention und/oder Behandlung von Viruskrankheiten.

25. Verwendung eines Oligoribonucleotids gemäß einem der Ansprüche 1 bis 9, eines Vorläufers gemäß Anspruch 10, eines Expressionsvektors gemäß Anspruch 12 oder Anspruch 13, Eukaryoten- oder Prokaryotenzellen gemäß Anspruch 14 oder eines nichtmenschlichen transgenen Tiers gemäß Anspruch 15 für die Targetierung von Molekülen, die fähig sind, die Aktivität der α-, α'- oder β-Untereinheiten der CK2-Proteinkinase zu modulieren.

## Claims

1. A double-stranded oligonucleotide made up of two strands of 1+9 to 23 nucleotides, each strand consisting, from 5' to 3', of a sequence of 17 to 21 ribonucleotides and two deoxyribo- or ribonucleotides, the 17 to 21 ribonucleotide RNA sequences of said strands being complementary and the two nucleotides of the 3' ends being protruding, **characterized in that** the RNA sequence of the sense strand or positive strand is that of a fragment of a transcript of an α, α' or β subunit of a CK2 protein kinase, selected from the group consisting of:
a) a fragment corresponding to an oligonucleotide which inhibits more than 80% of the expression of the corresponding subunit, in cell culture, at a concentration of between 1 and 200 nM, preferably less than 20 nM,
b) a fragment of a transcript of an α subunit included between positions 18-74, 259-279, 565-585, 644-664, 720-750, 808-831 and 863-885, from the ATG codon, with reference to the cDNA sequence of the CK2 α subunit of mouse No. NM_007787 or human No. NM_001895,
c) a fragment of a transcript of an α' subunit included between positions 49-69, 132-142, 306-326, 367-387, 427-447, 451-471, 595-615, 735-755, 827-847, 868-888, 949-969 and 988-1008, from the ATG codon, with reference to the cDNA sequence of the CK2 α' subunit of mouse NM_009974 or human No. NM_001896,
d) a fragment of a transcript of a β subunit included between positions 80-100, 116-137, 164-208, 369-389, 400-420, 527-591 and 613-643, from the ATG codon, with reference to the cDNA sequence of the CK2 β subunit of human No. NM_001320 or of mouse No. NP_034105, and
e) a fragment of 17 to 21 bases exhibiting at least 80% identity with the fragments defined in a), b), c) and d).

2. The double-stranded oligonucleotide as claimed in claim 1, **characterized in that** said 17 to 27 ribonucleotide RNA sequence of the sense strand is selected from the group consisting of:
a) a fragment of an α subunit defined by the RNA equivalent of the sequence SEQ ID Nos: 1 to 13,
b) a fragment of an α' subunit defined by the RNA equivalent of the sequence SEQ ID Nos: 14 to 25,
c) a fragment of a β subunit defined by the RNA equivalent of the sequence SEQ ID Nos: 26 to 40, and
d) a sequence as defined in a), b) or c), truncated by one or two ribonucleotides at its 5' and/or 3' end.

3. The double-stranded oligonucleotide as claimed in claim 1 or claim 2, **characterized in that** each of the strands comprises a phosphate group in the 5' position and a hydroxyl group in the 3' position.

4. The double-stranded oligonucleotide as claimed in any one of claims 1 to 3, **characterized in that** said protruding nucleotides of the 3' ends are selected from the group consisting of the pairs tt and aa.

5. The double-stranded oligonucleotide as claimed in any one of claims 1 to 4, **characterized in that** it is made up of two strands of 19 or 20 nucleotides.

6. The double-stranded oligonucleotide as claimed in claim 5, **characterized in that** the sense strand is defined by the sequence SEQ ID No. 67 or 68.

7. The double-stranded oligonucleotide as claimed in any one of claims 1 to 4, **characterized in that** it is made up of two strands of 21 to 23 nucleotides.

8. The double-stranded oligonucleotide as claimed in claim 7, **characterized in that** the sense strand is defined by the sequence SEQ ID Nos. 41 to 66, 69 to 81, 83 and 86.

9. The oligonucleotide as claimed in any one of claims 1 to 8, **characterized in that** it is stabilized.

10. A precursor of the oligonucleotide as claimed in any one of claims 1 to 9, **characterized in that** it is selected from the group consisting of:
a) a single-stranded oligonucleotide consisting of the sense or antisense strand of the oligonucleotide as claimed in any one of claims 1 to 9,
b) a double-stranded oligodeoxynucleotide corresponding to the sense and/or antisense strands of the oligonucleotide as claimed in any one of claims 1 to 9,
c) a hairpin oligoribonucleotide comprising the sequences of the sense and antisense strands of the double-stranded oligonucleotide as claimed in any one of claims 1 to 9,
d) a double-stranded oligodeoxynucleotide made up of a sense strand corresponding to the oligonucleotide defined in c) and of an antisense strand complementary thereto.

11. An expression cassette, **characterized in that** it comprises at least one precursor as defined in claim 10, under the control of appropriate transcriptional regulatory elements.

12. An expression vector, **characterized in that** it comprises the cassette as defined in claim 11.

13. The expression vector as claimed in claim 12, **characterized in that** it is a DNA vector comprising a DNA precursor as defined in b) and d) of claim 10 included in an expression cassette.

14. A eukaryotic or prokaryotic cell, **characterized in that** it is modified with an oligonucleotide as claimed in any one of claims 1 to 9, a precursor as claimed in claim 10, an expression cassette as claimed in claim 11 or an expression vector as claimed in claim 12 or claim 13.

15. A transgenic nonhuman animal, **characterized in that** it comprises cells modified with a precursor as claimed in claim 10, an expression cassette as claimed in claim 11 or an expression vector as claimed in claim 12 or claim 13.

16. A pharmaceutical composition, **characterized in that** it comprises at least one oligonucleotide as claimed in any one of claims 1 to 9, one precursor as claimed in claim 10 or one expression vector as claimed in claim 12 or claim 13, and a pharmaceutically acceptable carrier.

17. The pharmaceutical composition as claimed in claim 16, **characterized in that** said oligonucleotide, precursor or vector is associated with at least one substance that makes it possible to cross the plasma membrane.

18. The pharmaceutical composition as claimed in claim 16 or claim 17, **characterized in that** said oligonucleotide, precursor or vector is associated with at least one substance that allows targeting into cells, tissues or organs.

19. The pharmaceutical composition as claimed in any one of claims 16 to 18, **characterized in that** said oligonucleotide, precursor or vector is combined with at least one antiviral or anticancer agent.

20. The pharmaceutical composition as claimed in any one of claims 16 to 19, **characterized in that** it comprises a mixture of several oligonucleotides or of their precursors, or else one or more expression vectors for said mixture of oligonucleotides, in particular a mixture comprising at least one oligonucleotide specific for the α subunit, at least one oligonucleotide specific for the α' subunit and at least one oligonucleotide specific for the β subunit.

21. The use of an oligonucleotide as claimed in any one of claims 1 to 9, of a precursor as claimed in claim 10 or of an expression vector as claimed in claim 12 or claim 13, for the preparation of a medicament for use in the prevention and/or treatment of cancer.

22. The use of an oligonucleotide as claimed in any one of claims 1 to 9, of a precursor as claimed in claim 10 or of an expression vector as claimed in claim 12 or claim 13, for the preparation of a medicament for use in the prevention and/or treatment of viral diseases.

23. A product containing at least one oligonucleotide as claimed in any one of claims 1 to 9, one precursor as claimed in claim 10 or one expression vector as claimed in claim 12 or claim 13 and an anticancer active ingredient, as a combined preparation for simultaneous, separate or sequential use, in the prevention and/or treatment of cancer.

24. A product containing at least one oligoribonucleotide as claimed in any one of claims 1 to 9, one precursor as claimed in claim 10 or one expression vector as claimed in claim 12 or claim 13 and an antiviral active ingredient, as a combined preparation for simultaneous, separate or sequential use, in the prevention and/or treatment of viral diseases.

25. The use of an oligoribonucleotide as claimed in any one of claims 1 to 9, of a precursor as claimed in claim 10, of an expression vector as claimed in claim 12 or claim 13, of eukaryotic or prokaryotic cells as claimed in claim 14 or of a transgenic nonhuman animal as claimed in claim 15, for screening for molecules capable of modulating the activity of the α, α' or β subunits of the CK2 protein kinase.
